Europäisches Patentamt

European Patent Office

Office européen des brevets

(11) Publication number: **0 619 122 A1**

# EUROPEAN PATENT APPLICATION

(21) Application number: **93105876.2**

(22) Date of filing: **08.04.93**

(51) Int. Cl.5: **A61M 5/145**, H02P 8/00

(43) Date of publication of application:
**12.10.94 Bulletin 94/41**

(84) Designated Contracting States:
**DE FR IT**

(71) Applicant: **GETZ BROS. CO.,LTD.**
**Sumitomo Seimei Aoyama Bldg.,**
**3-1-30, Minami-Aoyama**
**Minato-ku, Tokyo 107 (JP)**

(72) Inventor: **Mizoguchi, Kazuaki, c/o Getz Bros.,**
**Ltd., Sumitomo**
**Seimei Aoyama Bldg.,**
**3-1-30, Minami-Aoyama**
**Minato-ku, Tokyo 107 (JP)**

(74) Representative: **Patentanwälte Liedl, Liedl,**
**Fritsche**
**Herterichstrasse 18**
**D-81479 München (DE)**

(54) **Syringe control system for DSA and PTCA.**

(57) A syringe (5) used in DSA (Digital Subtraction Angiography) and PTCA (Percutaneous Transluminal Coronary Angiography) is provided with a syringe drive unit driven by a motor (61). The motor velocity is controlled by a manual controlled variable voltage (71). A time sequence of the variable voltage which has given a successful control is stored in a memory. When a similar syringe control is requested, the stored data is read out from the memory to produce a control voltage of the motor (61).

FIG.1

## BACKGROUND OF THE INVENTION

The present invention relates to a syringe control system for DSA(Digital Subtraction Angiography) and PTCA(Percutaneous Transluminal Coronary Angiography).

Diagnosis by DSA and medical treatment by PTCA have become popularized in these years. Throughout these medical operations of DSA and PTCA, the operated portion is observed by an X-ray monitor television display. In order to obtain a clear view of the operated portion on the display, contrast medium must be injected in blood vessels in the operated portion. And syringe manipulation for injecting the contrast medium has been a burden to the operator.

In one example of PTCA, a guide catheter holding a balloon catheter within, is first inserted into the lumen of the coronary artery, and subsequently, a guide wire going through the through lumen of the balloon catheter is pushed through a target coronary artery. A balloon which is formed at an end of the balloon catheter is pushed along the guide wire to be placed in the stenosed coronary artery lumen. The balloon is then inflated to expand the stenosed portion.

Throughout these operations of pushing the guide wire and the balloon catheter, contrast medium must be injected in the lumen, and since the contrast medium is dispersed in a short time, the injection must be so repeated that a clear display is obtained for each observation.

For the injection of contrast medium, a syringe is pushed little by little by manually adjusting the push amount to inject contrast medium through the aperture between the guide catheter and the balloon catheter.

The manipulation of pushing the syringe for contrast medium injection requires a very fine adjustment, and the pressure injection of the contrast medium requires a relatively large force for a hand-exerted force. Moreover, a similar manipulation must be repeated over and over again.

Thus, the syringe manipulation has been a burden which gives the operator an extreme tension and exhaustion.

And, the operator is exposed to a fairly large amount of radioactivity, as the operator must remain close by the patient.

## SUMMARY OF THE INVENTION

Therefore, an object of this invention is to provide a power-drive for a syringe, in which the drive unit is controlled through a hand-control or a foot-control. An electric circuit translates a fairly large displacement in the hand- or foot-control to a very fine displacement of the syringe piston. And, since the syringe piston is displaced by a motor of the syringe drive unit, the high pressure exerted on the syringe piston has no relation to the force for driving the hand-control or the foot-control.

Another object of this invention is to provide means for controlling the syringe drive unit by stored data which have given a successful result in a preceding control. By this stored data control, worrisome repetitions of similar manipulation are eliminated.

Still another object of this invention is to place the hand- or foot-control at a distance from the motor-driven syringe in order to protect the operator from the radioactivity.

These and other objects, features and advantages of the present invention will become clear from the following description of the preferred embodiments in conjunction with the accompanying drawings in which the same numerals indicate the same or the corresponding parts.

## BRIEF DESCRIPTION OF THE DRAWINGS

Fig. 1 shows a block diagram of an embodiment of the present invention.

Fig. 2 shows a crosssectional view of the guide catheter and the balloon catheter in Fig. 1.

Figs. 3 show schematic views of guide catheter insertion and balloon catheter pushing.

Fig. 4 shows an example of the control unit in Fig. 1.

Fig. 5 shows another example of the control unit in Fig. 1.

Fig. 6 shows a modification of Fig. 5.

Fig. 7 shows a block diagram of the host computer in Fig. 1.

Fig. 8 shows an example of injection control.

Fig. 9 shows an example of replenishment control.

## DETAILED DESCRIPTION OF THE PREFERRED EMBODIMENTS

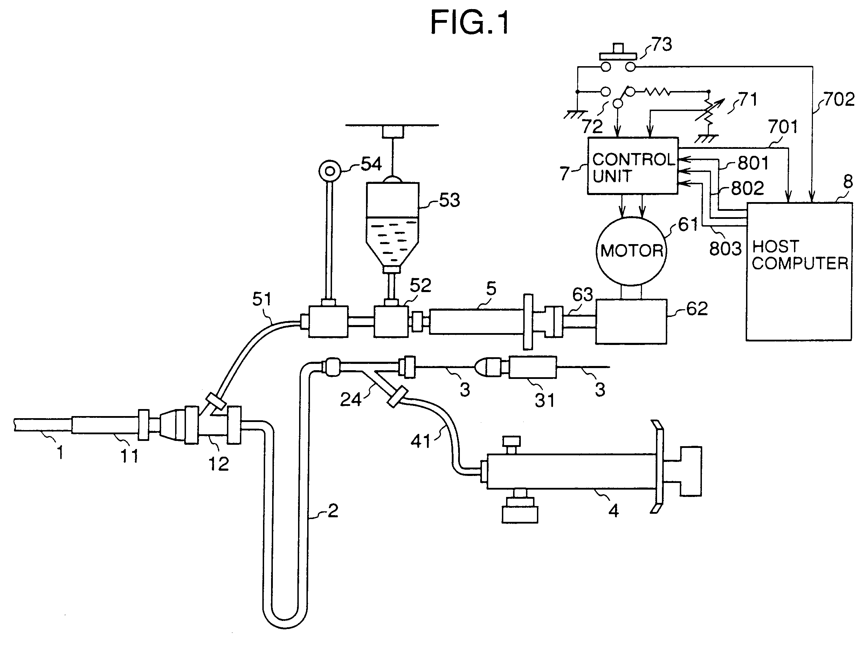

Referring to Fig. 1, devices related to this invention will be explained in connection with PTCA. The near end of a guide catheter 1 is fastened in a Y-connector 12. The far end of a balloon catheter 2 is inserted through a leg of the Y-connector 12 into the lumen of the guide catheter 1.

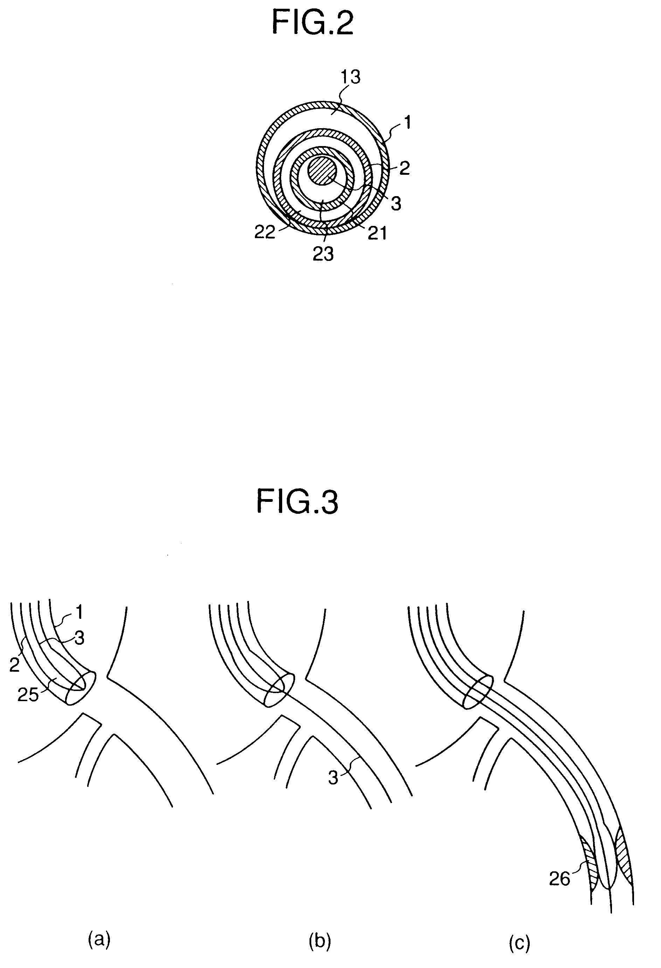

Referring now to the crosssectional view of Fig. 2, the balloon catheter 2 is held in the guide catheter 1, and a lumen 13 between the guide catheter 1 and the balloon catheter 2 forms a duct for contrast medium. The balloon catheter 2 has an inner tube 21 which holds a guide wire 3 within. The lumen 22 between the balloon catheter 2 and the inner tube 22 is called a deflation/inflation lumen. The lumen 23 in the inner tube 21 is called a through lumen. The far end of the balloon catheter 2 is formed into a balloon(not shown in the drawing).

Returning to Fig. 1, a tube 51 which conveys contrast medium from a syringe 5, is connected to the lumen 13 through another leg of the Y-connector 12. The near end of the balloon catheter 2 is fastened in another Y-connector 24. A guide wire 3 is inserted from one leg of the Y-connector 24 into the through lumen 23 of the balloon catheter 2. A tube 41 which conveys inflation medium from an inflator/deflator 4, is connected to the deflation/inflation lumen 22 through another leg of the Y-connector 24.

A torquer 31 is to push the guide wire 3 through the through lumen 41 of the balloon catheter 2. The piston(not shown in the drawing) of the syringe 5 is driven by a driving rod 63. When the cylinder of the syringe 5 is empty, the contrast medium is replenished automatically from a contrast medium reservoir 53 through an automatic operated three way cock valve 52. The pressure of the outgoing contrast medium is indicated by a pressure gauge 54.

A gear mechanism 62 translates the rotational motion of a polyphase stepping motor 61 to a rectilinear motion of the driving rod 63. The speed of the motor 61 is controlled by the frequency of the voltage from a control unit 7, and the direction of motor rotation is determined by the phase relation of the polyphase voltage from the control unit 7. A manual control 71 controls the frequency of the output voltage of the control unit 7, and a binary signal from a changeover switch 72 determines the phase relation of the output voltage of the control unit 7.

Essential data in the control unit 7 are input through data bus 701 to a host computer 8 and are stored. When a push button switch 73 is pushed, a signal is delivered to the host computer 8. In response to this signal, the host computer 8 starts to read out the stored data on data bus 801 and transmits a command signal on a control line 802. The command signal on the control line 802 changes the circuit in the control unit 7 to generate the output voltages from data on the data bus 801.

A clock line 803 transmits a clock signal from the host computer 8 to the control unit 7, where the clock signal is used as a sampling clock in an analog to digital converter. Since this clock signal is used as a memory writing and reading clock in the host computer 8, the bit rate of the read out data on the data bus 801 is equal to that on the data bus 701.

In an operation, a sheath 11 is inserted into a femoral vein(in case of a transfemoral method), and the guide catheter 1 holding the balloon catheter 2 within, is pushed through the sheath 11 and the femoral vein to an orifice of a target coronary artery. Fig. 3(a) shows this phase, where a balloon 25 which is formed at the far end of the balloon catheter 2 is within the guide catheter 1. At this situation, the manual control 71 is controlled to give the driving rod 63 a forward(determined by the position of the changeover switch 72)motion at a controlled speed. The contrast medium in the syringe 5 is pushed and an amount of the contrast medium flows out from the far end of the lumen 13. The flowed out contrast medium enters in neighboring blood vessels making the blood vessels observable on the X-ray monitor display.

Watching the display, the operator handles the torquer 31 to push the guide wire 3 to proceed in a target artery. This phase is shown in Fig. 3(b). When the guide wire 3 has been passed through a stenosed portion 26 of the lumen, the balloon catheter 2 is manually pushed through the Y-connector 12. The balloon catheter 2 is advanced in the artery guided by the guide wire 3 to reach at the stenosed portion 26. This phase is shown in Fig. 3-(c).

At the situation shown in Fig. 3(c), the inflator/deflator 4 is manipulated, and a medium is forced through the deflation/inflation lumen 22 to inflate the balloon 25 and expand the stenosed portion 26.

The pressure of injection indicated by the pressure gauge 8 must be within a certain limit, lest the pressurized medium should harm a weakened artery. And a total amount of a dose must be limited under a certain quantity in order to avoid harmful side effect. For example, in the so-called test shot which is injected for watching the path of the guide wire 3 and the balloon catheter 2, the total amount of a dose is 2~3cc, and in the so-called control shot which is injected for observing the expanded stenosed portion 26, the total amount of a dose is 7~8cc. The controlled shot must also be repeated several times for observations from several different directions.

The syringe control system of this invention is best adapted to be used in these delicately controlled repeated manipulations in order to remove the burden from the operator.

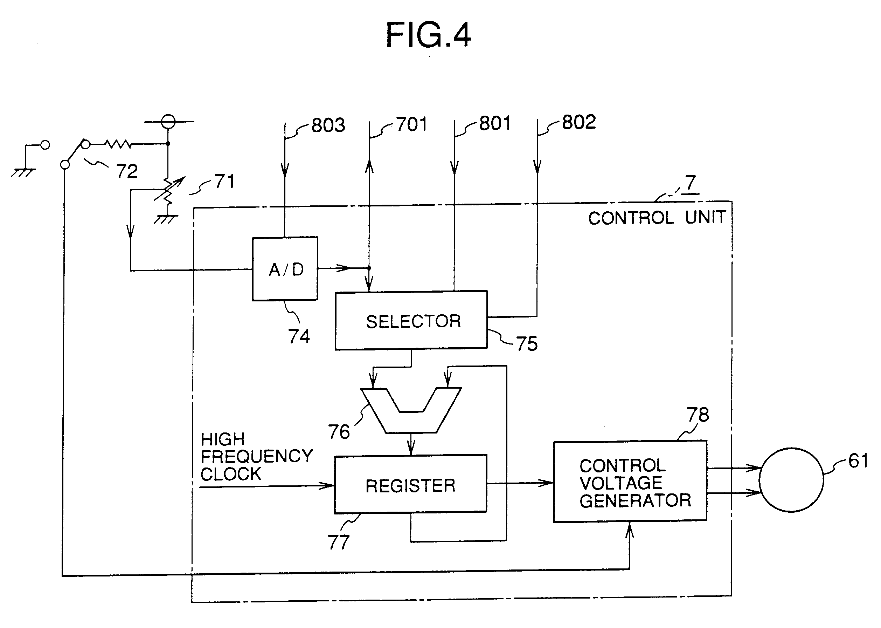

Fig. 4 shows a block diagram of an embodiment of the control unit 7 of this invention. A controlled voltage E from the manual control 71 is converted to a digital number representing the magnitude of E by an analog to digital converter-(A/D) 74. The sampling at the A/D 74 is controlled by the clock signal delivered from the host computer 8 on the clock line 803. The digital number E is transmitted on the data bus 701 to the host computer 8 and is stored in a memory. The same digital number E read out from the memory is delivered to the control unit 7 from the host computer 8 on the data bus 801. A selector 75 is controlled by the signal on the control line 802.

In a manual control mode, the selector 75 connects the output E of the A/D 74 to an input of a digital adder 76. To another input of the adder 76 is connected the content of a register 77. When the content of the register is U, the output of the adder 76 is $U + E$, and this output is connected to the input terminal of the register 77. When a pulse of a high frequency clock is applied to the loading terminal of the register 77, the signal at the input of the register 77, which at this time is $U + E$, is loaded to the register 77, and the content of the register 77 is increased to $U + E$. Thus, for each pulse of the high frequency clock, the content of the register 77 is increased by E. When the frequency of the high frequency clock is F Hertz, the content of the register 77 is increased by $E \times F$ in a second. When the modulo of the register 77 is M, a carry-over pulse having a frequency of $(E \times F)/M$ overflows from the register 77. This carry-over pulse having a frequency proportional to the controlled voltage E, determines the frequency of the polyphase rectangular waveform voltage generated in a control voltage generator 78. The phase rotation of the generated polyphase voltage is determined by the logic of the output from the changeover switch 72.

The adder 76 and the register 77 together with the high frequency clock, constitute a pulse generating circuit for generating a pulse train having a frequency proportional to an input digital number, and it must be noted that any other pulse generating circuit for generating a pulse train having a frequency proportional to an input digital number, may be used in this invention.

During the manual control mode, the digital number on the data bus 701 is stored in the host computer 8. When a manual control mode is succeeded by another manual control mode, the data stored in the preceding manual control mode may be erased. When a successful result has been obtained by a manual control mode and the operator desires to repeat the successful control, the push button switch 73(Fig. 1) is pushed. A signal requesting a memory control mode is transmitted to the host computer 8. In response to the request signal, the host computer 8 transmits a control signal on the control line 802, and reads out the stored data of the successful manual control on the data bus 801.

The control signal on the control line 802 controls the selector 75, and the data on the data line 801 is connected to one input of the adder 76. And thus, the control voltage generator 78 is controlled by the stored data of the successful manual control.

The remaining volume of the contrast medium in the syringe 5 is monitored, and when the volume becomes less than a limit predetermined for a case, the syringe 5 is replenished with contrast medium from the contrast medium reservoir 53. For this purpose, the three-way cock valve 52 is automatically operated to close the passage to the tube 51 and open the passage to the reservoir 53. The changeover switch 72 is operated automatically or manually to change the direction of rotation of the motor 61. Then the manual control 71 is controlled to drive the driving rod 63 in a reversed direction to suck the contrast medium from the reservoir 53 to the syringe 5.

The replenishment of the syringe 5 may also be memory controlled. And since the motion of the cylinder of the syringe 5 can take a same pattern in all the replenishment control, this pattern of motion is stored beforehand in the memory and is read out on the data bus 801 after the changeover of the three-way cock valve 52 is completed.

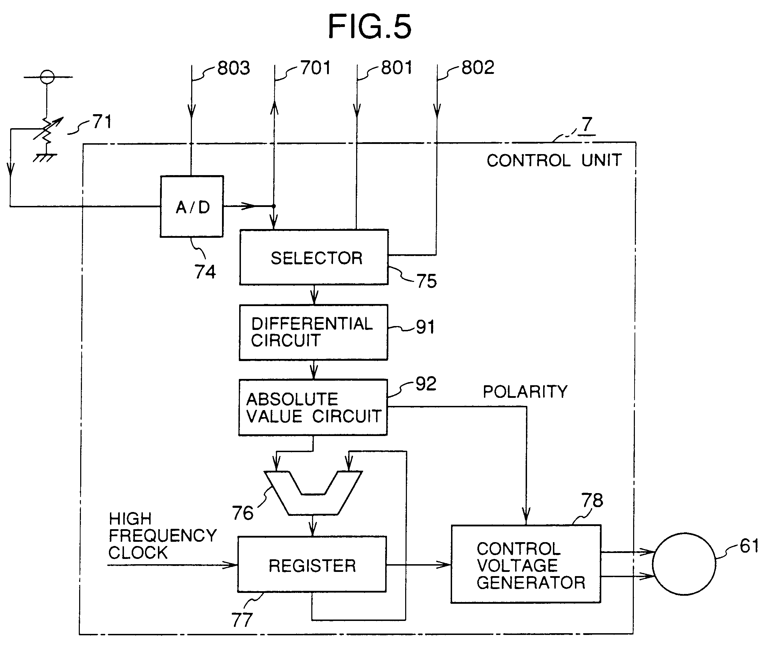

Fig. 5 shows another embodiment of the control unit 7 in Fig. 1. In this embodiment, a differential circuit 91 produces a time derivative of the digital number E, that is, $dE/dt$. Although E is a positive number, $dE/dt$ is positive for an increasing E and negative for a decreasing E, and a negative number is represented by a sign bit and a complementary number in the output of the differential circuit 91. This representation of a digital number is transformed into a polarity bit and an absolute value of the digital number in an absolute value circuit 92.

The absolute value of $dE/dt$ determines the speed of the motor 61 and the polarity of $dE/dt$ determines the direction of the rotation of the motor 61. When a displacement of the driving rod 63 is denoted by s, its velocity(speed and direction) is $ds/dt$. Since the velocity of the driving rod 63 is proportional to the velocity of the motor 61, which is proportional to $dE/dt$, $ds/dt = kdE/dt \cdots (1)$. Integrating the equation(1) $s = s_0 + kE \cdots (2)$ is obtained where $s_0$ is an integration constant.

Thus, the variable voltage E controls the speed of the driving rod 63 in the embodiment of Fig. 4, and controls the displacement of the driving rod 63 in the embodiment of Fig. 5.

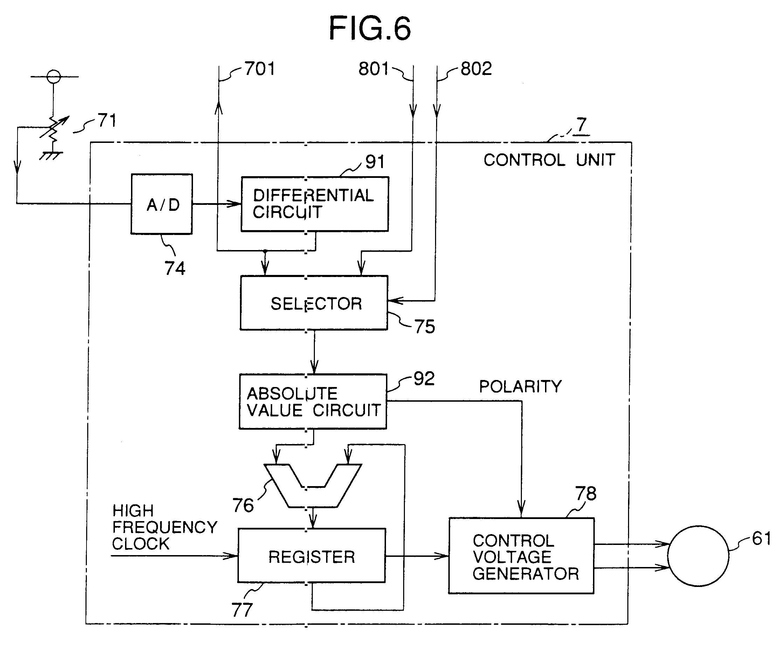

Fig. 6 shows a modification of the control unit of Fig. 5. In this modification shown in Fig. 6, the output of the differential circuit 91, that is $dE/dt$, is transmitted on the data bus 701 to the host computer, and $dE/dt$ read out from the memory is delivered on the data bus 801 as one input of the selector 75.

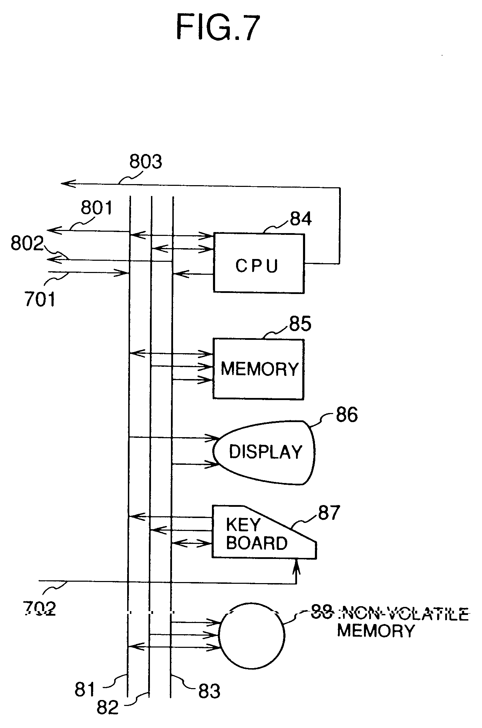

Fig. 7 shows a block diagram of the host computer 8 in Fig. 1. A common data bus 81, a common address bus 82, and a common control bus 83 interconnect a CPU 84, a memory 85, a display 86, a keyboard 87, and a non-volatile memory 88. The data from the control unit 7 is stored in the memory 85, and the data read out from the memory 85 is transmitted to the control unit 7. When required, the data in the memory 85 is transferred to the non-volatile memory 88 and is stored there.

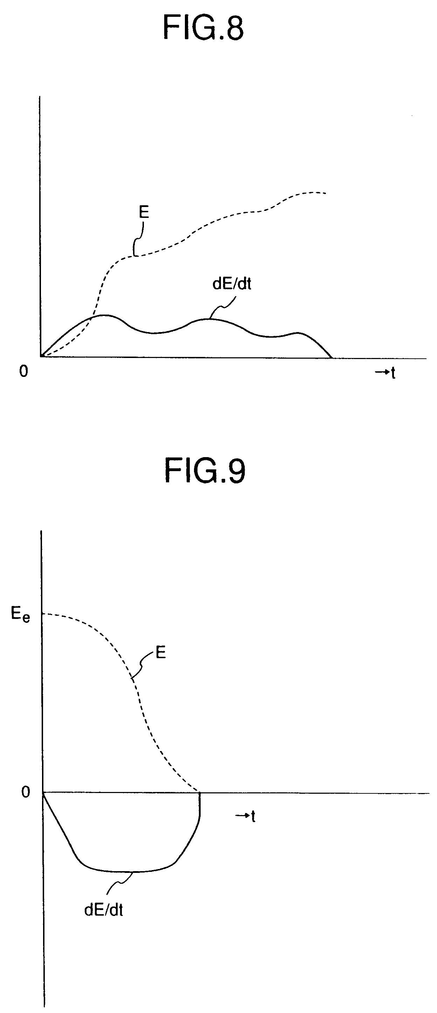

Fig. 8 shows an example of data transmitted on the data bus 801 in an injection control. Digital number representing E is transmitted in an embodiment shown in Fig. 4 or in Fig. 5, and digital number representing dE/dt is transmitted in an embodiment shown in Fig. 6.

Fig. 9 shows an example of data transmitted on the data bus 801 in a replenishment control.

Although only preferred embodiments are described in this specification, it should be understood that various changes and modifications may be made without departing from the spirit of this invention.

## Claims

1. Syringe control system for DSA and PTCA, comprising:

a syringe(5) for injecting contrast medium into an artery lumen to be observed by an X-ray monitor television display, through a lumen(13) formed between a guide catheter and a balloon catheter which is inserted in said guide catheter,

a driving rod(63) connected to the piston of said syringe,

a gear mechanism(62) for driving said driving rod in a forward

direction or in a backward direction translating a rotational motion of a motor, a polyphase stepping motor(61) engaged to said gear mechanism,

a manual control(71) for producing a variable voltage,

an analog to digital converter(74) for producing a digital number representing said variable voltage,

means for storing said digital number in a memory(85),

means for reproducing said digital number from said memory,

a selector(75) for selecting either said digital number produced by said analog to digital converter or said digital number reproduced from said memory,

a pulse generating circuit(76, 77) for generating a pulse train having a frequency proportional to said digital number selected by said selector,

a control voltage generator(78) for generating a polyphase rectangular waveform voltage having a frequency determined by the frequency of said pulse train generated by said pulse generating circuit, and

means for applying said polyphase rectangular waveform voltage to said polyphase stepping motor.

2. Syringe control system for DSA and PTCA, comprising:

a syringe(5) for injecting contrast medium into an artery lumen to be observed by an X-ray monitor television display, through a lumen(13) formed between a guide catheter and a balloon catheter which is inserted in said guide catheter,

a driving rod(63) connected to the piston of said syringe,

a gear mechanism(62) for driving said driving rod in a forward direction or in a backward direction translating a rotational motion of a motor,

a polyphase stepping motor(61) engaged to said gear mechanism,

a manual control(71) for producing a variable voltage,

an analog to digital converter(74) for producing a digital number representing said variable voltage,

means for storing said digital number in a memory(85),

means for reproducing said digital number from said memory,

a selector(75) for selecting either said digital number produced by said analog to digital converter or said digital number reproduced from said memory,

a differential circuit(91) for producing a time derivative of said selected digital number,

a pulse generating circuit(76, 77) for generating a pulse train having a frequency proportional to an absolute value of said time derivative of said selected digital number,

a control voltage generator(78) for generating a polyphase rectangular waveform voltage having a frequency determined by the frequency of said pulse train generated by said pulse generating circuit, and

means for applying said polyphase rectangular waveform voltage to said polyphase stepping motor.

3. Syringe control system for DSA and PTCA, comprising:

a syringe(5) for injecting contrast medium into an artery lumen to be observed by an X-ray monitor television display, through a lumen(13) formed between a guide catheter and a balloon catheter which is inserted in said guide catheter,

a driving rod(63) connected to the piston of said syringe,

a gear mechanism(62) for driving said driving rod in a forward direction or in a backward direction translating a rotational motion of a motor,

a polyphase stepping motor(61) engaged to said gear mechanism,

a manual control(71) for producing a variable voltage,

means(74, 91) for producing a digital number representing a time derivative of said variable voltage,

means for storing said digital number in a memory(85),

means for reproducing said digital number from said memory,

a selector(75) for selecting either said digital number produced by said means for producing a digital number or said digital number reproduced from said memory,

a pulse generating circuit(76, 77) for generating a pulse train having a frequency proportional to an absolute value of said digital number selected by said selector,

a control voltage generator(78) for generating a polyphase rectangular waveform voltage having a frequency determined by the frequency of said pulse train generated by said pulse generating circuit, and

means for applying said polyphase rectangular waveform voltage to said polyphase stepping motor.

FIG.1

# FIG.2

# FIG.3

(a)

(b)

(c)

# FIG.4

803　701　801　802

CONTROL UNIT 7

A/D 74

SELECTOR 75

76

HIGH
FREQUENCY
CLOCK

REGISTER 77

CONTROL
VOLTAGE
GENERATOR 78

61

72　71

EP 0 619 122 A1

# FIG.5

CONTROL UNIT 7

A/D 74

SELECTOR 75

DIFFERENTIAL CIRCUIT 91

ABSOLUTE VALUE CIRCUIT 92

POLARITY

HIGH FREQUENCY CLOCK

REGISTER 77

76

CONTROL VOLTAGE GENERATOR 78

61

71

803 701 801 802

EP 0 619 122 A1

FIG.6

CONTROL UNIT 7

A/D 74
DIFFERENTIAL CIRCUIT 91
SELECTOR 75
ABSOLUTE VALUE CIRCUIT 92
REGISTER 77
CONTROL VOLTAGE GENERATOR 78
POLARITY
HIGH FREQUENCY CLOCK
76
701, 801, 802, 71, 61

11

# FIG.7

# FIG.8

E

dE/dt

0

→t

# FIG.9

$E_e$

E

0

→t

dE/dt

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y | DE-A-2 529 636 (JUNGINGER) <br> * page 2, line 26 - page 3, line 3; figure * | 1 | A61M5/145 <br> H02P8/00 |
| Y <br><br> A | EP-A-0 199 613 (CENTRE TECHNIQUE DES INDUSTRIES MECHANIQUES) <br> * column 3, line 32 - line 50 * <br> * column 9, line 8 - line 16 * <br> * column 9, line 41 - column 10, line 28; figures 3-5,11 * | 1 <br><br><br><br><br> 2,3 | |
| A | WO-A-8 502 546 (DISETRONIC AG) <br> * page 4, line 11 - line 12 * <br> * page 7, line 19 - line 22 * <br> * page 12, line 23 - page 13, line 23; figure * | 1-3 | |
| A | US-A-4 714 867 (PALMIN ET AL.) <br> * column 2, line 29 - line 38; figures 3,4 * | 1-3 | |
| A | WO-A-8 202 127 (IVAC CORPORATION) <br> * page 5, line 7 - line 11 * <br> * page 11, line 22 - line 27; figures 1,3 * | 1-3 | TECHNICAL FIELDS SEARCHED (Int. Cl.5 ) <br><br> A61M |
| A | EP-A-0 465 267 (SEIKO EPSON CORPORATION) <br> * column 6, line 48 - column 7, line 3; figure 11 * | 1-3 | |

-----

The present search report has been drawn up for all claims

| Place of search | Date of completion of the search | Examiner |
|---|---|---|
| THE HAGUE | 16 SEPTEMBER 1993 | SEDY R. |